(19) Europäisches Patentamt
European Patent Office
Office européen des brevets

(11) Veröffentlichungsnummer: **0 034 754**
**A1**

(12) # EUROPÄISCHE PATENTANMELDUNG

(21) Anmeldenummer: **81100948.9**

(22) Anmeldetag: **11.02.81**

(51) Int. Cl.³: **C 07 D 261/08**
C 07 D 261/10, C 07 D 261/18
A 61 K 31/42
//C07F9/65, C07D401/12

(30) Priorität: **23.02.80 DE 3006809**

(43) Veröffentlichungstag der Anmeldung:
**02.09.81 Patentblatt 81/35**

(84) Benannte Vertragsstaaten:
**AT BE CH DE FR GB IT LI LU NL SE**

(71) Anmelder: **BASF Aktiengesellschaft**
**Carl-Bosch-Strasse 38**
**D-6700 Ludwigshafen(DE)**

(72) Erfinder: **Schlecker, Rainer, Dr.**
**Suedring 8**
**D-6719 Bissersheim(DE)**

(72) Erfinder: **Frickel, Fritz-Frieder, Dr.**
**Silvanerweg 7**
**D-6705 Deidesheim(DE)**

(72) Erfinder: **Franke, Albrecht, Dr.**
**Mandelring 11**
**D-6706 Wachenheim(DE)**

(72) Erfinder: **Lenke, Dieter, Dr.**
**Kekuleplatz 1**
**D-6700 Ludwigshafen(DE)**

(72) Erfinder: **Gries, Josef, Dr.**
**Roemerweg 43**
**D-6706 Wachenheim(DE)**

(54) **2-((3-Amino-2-hydroxy-propoxyl)-styryl)-isooxazole, Verfahren zu ihrer Herstellung und diese enthaltende pharmazeutische Zubereitungen.**

(57) Gegenstand der vorliegenden Erfindung sind Aminopropanol-äther von 2-Hydroxystyryl-isoxazolen und ihre physiologisch verträglichen Säureadditionssalze, Verfahren zu ihrer Herstellung und diese Verbindungen enthaltenden pharmazeutischen Zubereitungen, die als wertvolle Arzneimittel bei der Behandlung der Hypertonie, der koronaren Herzkrankheit und von Herzrhythmusstörungen verwendet werden können.

EP 0 034 754 A1

Croydon Printing Company Ltd.

2-[(3-Amino-2-hydroxy-propoxy)-styryl]-isoxazole, Verfahren zu ihrer Herstellung und diese enthaltende pharmazeutische Zubereitungen

Gegenstand der vorliegenden Erfindung sind Aminopropanoläther von 2-Hydroxystyryl-isoxazolen und ihre physiologisch verträglichen Säureadditionssalze, Verfahren zu ihrer Herstellung und diese Verbindungen enthaltenden pharmazeutischen Zubereitungen, die als wertvolle Arzneimittel bei der Behandlung der Hypertonie, der koronaren Herzkrankheit und von Herzrhythmusstörungen verwendet werden können.

Aus der DE-OS 28 18 999 ist bekannt, daß Alkylaminopropanoläther des 3-Alkyl-5-(2-hydroxystyryl)-isoxazols als ß-Sympatholytika mit blutdrucksenkender Wirkung und aufgrund dieses Effektes bei der Hypertonie, der koronaren Herzkrankheit und Herzrhythmusstörungen verwendet werden können. Die gefundenen Wirkungen befriedigen nicht immer.

Es wurde gefunden, daß Verbindungen der allgemeinen Formel I

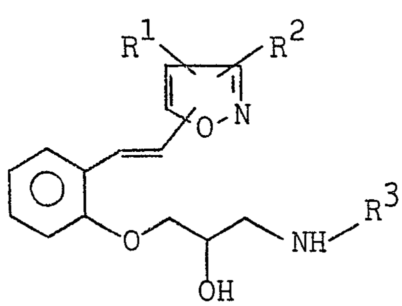

(I)

in der $R^1$ und $R^2$ gleich oder verschieden sind und ein Wasserstoffatom, einen Alkylrest mit 1 bis 6 C-Atomen, der gegebenenfalls durch Hydroxy, Amino, Monoalkylamino, Dialkylamino oder Alkoxy mit jeweils 1 bis 3 C-Atomen im

D/BL

Alkyl substituiert ist, eine Nitro-, Cyano-, Carboxy-, Carboxamido-, Carbalkoxy- oder Carboxalkylamido-gruppe mit jeweils 1 bis 3 C-Atomen im Alkyl, ein Halogenatom, eine Hydroxy-, Amino-, Alkylamino-, Dialkylamino- oder Alkoxygruppe mit jeweils 1 bis 3 C-Atomen im Alkyl bedeuten, ausgenommen Verbindungen, bei denen im Isoxazolring der Styrylrest sich in 5-Stellung befindet und in 4-Stellung ein Wasserstoffatom und in 3-Stellung ein Alkylrest mit 1 bis 4 C-Atomen steht, und $R^3$ ein Wasserstoffatom, einen Alkylrest mit 1 bis 8 C-Atomen, der gegebenenfalls durch Hydroxy, Alkoxy mit 1 bis 3 C-Atomen im Alkyl oder einen Cycloalkylrest mit 3 bis 8 C-Atomen im Ring substituiert ist, einen Alkenyl- oder Alkinylrest mit 2 bis 8 C-Atomen, einen Cycloalkylrest mit 3 bis 8 C-Atomen im Ring, wobei die Cycloalkylringe gegebenenfalls ein- oder zweifach durch einen Alkylrest mit 1 bis 3 C-Atomen substituiert sind, darstellt, und ihren physiologisch verträglichen Säureadditionssalze, die wertvolle pharmakologische Eigenschaften aufweisen.

Als Alkylreste für $R^1$ und $R^2$ kommen insbesondere Reste mit 1 bis 4 C-Atomen, wie Methyl, Äthyl, Propyl, Isopropyl, n-Butyl, sek.-Butyl und tert.-Butyl, die gegebenenfalls durch Alkoxy mit 1 bis 3 C-Atomen substituiert sind, in Betracht. Davon sind Methyl, Äthyl und Methoxymethyl bevorzugt.

Als Alkylreste für $R^3$ mit 1 bis 8 C-Atomen, geradkettig oder verzweigt, seien Methyl, Äthyl, Propyl, Isopropyl, n-Butyl, sek.-Butyl, tert.-Butyl, Pentyl-2, 2-Methyl-butyl-2, 3-Methyl-butyl-2, 3-Methyl-pentyl-3, 2,3-Dimethyl-butyl-2, 3-Äthyl-pentyl-3, 2,4-Dimethyl-pentyl-3, 2,4,4-Trimethyl-pentyl und als substituierte Alkylreste 1-Methoxypropyl-2, 2-Hydroxyäthyl-1, 1-Hydroxybutyl-2, 3-Hydroxy-3-methyl-butyl-1, 1-Cyclopropyl-äthyl-1 genannt.

Von den Alkylresten sind solche mit 3 bis 6 C-Atomen und mit einer Verzweigung zum Aminstickstoff α-ständigen Kohlenstoffatom bevorzugt. Besonders bevorzugt sind Isopropyl und tert.-Butyl.

Als Alkenyl- oder Alkinylreste für $R^3$ mit 2 bis 8 C-Atomen seien 1-Propen-3-yl, 3-Butin-2-yl, 3-Methyl-3-butin-2-yl und 3-Äthyl-1-pentin-3-yl genannt. Davon ist 3-Methyl-1-butin-3-yl bevorzugt.

Cycloalkylreste sind beispielsweise Cyclopropyl, Cyclobutyl, Cyclopentyl, Cycloheptyl und Dimethylcyclohexyl, wobei als Alkylsubstituent für die genannten cyclischen Reste insbesondere Methyl in Betracht kommt. Von den Cycloalkylresten ist der Cyclopropylrest bevorzugt.

Der Isoxazolring weist den Styrylrest insbesondere in 3- oder 5-Stellung auf, wovon die 5-Stellung bevorzugt ist.

Die Verbindungen der Formel I können hergestellt werden, indem man einen Phenolether der Formel II

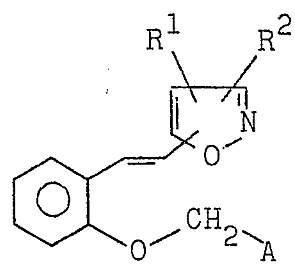

(II)

in der $R^1$ und $R^2$ die für Formel I angegebenen Bedeutungen haben und A den Rest

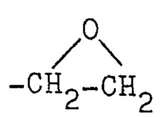 oder 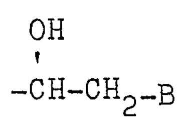

worin B für eine nukleofuge Abgangsgruppe steht, bedeutet, mit einem Amin der allgemeinen Formel

$$H_2N-R$$

in der R die für $R^3$ in Formel I angegebenen Bedeutungen hat, zweckmäßigerweise in einem Lösungsmittel und gegebenenfalls in Gegenwart eines säurebindenden Mittels in an sich bekannter Weise umsetzt und gegebenenfalls die erhaltene Verbindung in ein Säureadditionssalz einer physiologisch verträglichen Säure überführt.

Die Abgangsgruppe B stellt bevorzugt ein Halogenatom, insbesondere Chlor, Brom oder Jod, dar. Weiterhin kommen beispielsweise aromatische oder aliphatische Sulfonsäurereste wie der p-Toluolsulfonsäure-, der Brombenzolsulfonsäure- oder der Methansulfonsäurerest in Betracht.

Die Umsetzungen werden bei Temperaturen von 10 bis 120°C, d.i. bei Raumtemperatur oder bei höheren Temperaturen, zweckmäßig bei Temperaturen von 50 bis 120°C, unter Atmosphärendruck oder in einem geschlossenen Gefäß unter erhöhtem Druck durchgeführt.

Insbesondere bei leichtflüchtigen Aminen $H_2N-R$ kann es vorteilhaft sein, die Umsetzung in einem Autoklaven durchzuführen. Die Ausgangsverbindungen können direkt ohne Verdünnungs- oder Lösungsmittel umgesetzt werden. Zweckmäßigerweise werden die Umsetzungen jedoch durchgeführt in Gegenwart eines inerten Verdünnungs- oder Lösungsmittels, beispielsweise eines niederen Alkohols mit 1 bis 4 C-Atomen, wie Methanol, Äthanol oder Propanol, vorzugsweise Isopropanol oder Äthanol, eines niederen gesättigten Dialkyläthers, Dialkylglykoläthers oder cyclischen Äthers, wie Diäthyläther, 1,2-Dimethoxyäthan, Tetrahydrofuran oder

Dioxan, eines Benzolkohlenwasserstoffs, wie Benzol selbst oder eines Alkylbenzols, insbesondere Toluol oder Xylol, oder eines aliphatischen Kohlenwasserstoffs, wie Hexan, Heptan oder Octan, eines niederen aliphatischen Ketons, wie Aceton, Methyläthylketon oder Methyl-isobutylketon, eines Dialkylformamids, wie Dimethyl- oder Diäthylformamid, von Dimethylsulfoxid oder in Gegenwart von Wasser oder in Mischungen der genannten Lösungsmittel.

Auch ist das in überschüssiger Menge verwendete Amin der Formel $H_2N-R$ gegebenenfalls als Verdünnungs- oder Lösungsmittel geeignet.

Bevorzugte Lösungsmittel bei Umsetzungen von Verbindungen der Formel II, in denen A den Rest $\overbrace{-CH-CH_2}^{O}$ bedeutet, mit einem Amin $RNH_2$ sind niedere Alkohole, insbesondere Äthanol oder Isopropanol, wobei die Umsetzung bevorzugt bei Temperaturen von 50 bis 120°C durchgeführt wird. Gegebenenfalls kann die Umsetzung in einem geschlossenen Gefäß unter Druck durchgeführt werden, insbesondere wenn niedrigsiedende Amine eingesetzt werden. Bei der nukleophilen Substitution eines Restes B sind als Lösungsmittel ein niederes aliphatisches Keton, wie Aceton, Methyläthylketon oder Methylisobutylketon, ein cyclischer aliphatischer Äther, insbesondere Tetrahydrofuran oder Dioxan, oder ein Dialkylformamid, wie Dimethylformamid, und Temperaturen von 90°C bis 120°C bevorzugt. Gegebenenfalls wird die Reaktion in Gegenwart einer katalytischen Menge Natrium- oder Kaliumjodid durchgeführt. Die Ausgangsverbindung der Formel II kann gegebenenfalls auch in Form der bei ihrer Herstellung anfallenden Mischung aus Epoxid und Halogenhydrin umgesetzt werden.

Bei einer zweckmäßigen Ausführungsform zur nukleophilen Substitution des Restes B durch das verwendete Amin wird

die Umsetzung in Gegenwart einer Base als säurebindendes Mittel durchgeführt. Bevorzugte Basen sind Alkalimetallhydroxide, -carbonate, -hydrogencarbonate, -alkoholate, insbesondere Methylate und Äthylate, oder ein tertiäres organisches Amin, wie Pyridin, oder ein Trialkylamin, wie Trimethylamin oder Triäthylamin. Von den Alkaliverbindungen kommen insbesondere die des Natriums und Kaliums in Betracht. Dabei wird die Base in stöchiometrischer Menge oder in geringem Überschuß verwendet. Gegebenenfalls kann das zur Umsetzung verwendete Amin $R-NH_2$ im Überschuß gleichzeitig als säurebindendes Mittel verwendet werden.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z.B. durch Filtration oder Abdestillieren des Verdünnungsmittels aus dem Reaktionsgemisch. Eine Reinigung der erhaltenen Verbindung erfolgt in üblicher Weise, beispielsweise durch Umkristallisation aus einem Lösungsmittel, Überführen in eine Säureadditionsverbindung oder durch Säulenchromatographie.

Die Ausgangsverbindungen der Formel II können durch Alkylierung einer (2-Hydroxy-styryl)-Verbindung der Formel III,

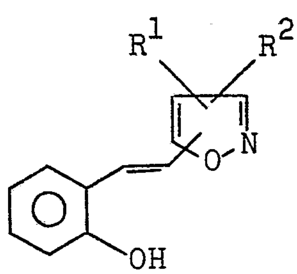

(III)

worin $R^1$ und $R^2$ die für Formel I angegebenen Bedeutungen haben, mit einem Epihalogenhydrin oder einem $\alpha,\omega$-Dihalogen-2-propanol erhalten werden. Als Epihalogenhydrine kommen Epichlorhydrin, Epibromhydrin und Epijodhydrin und als

α,ω-Dihalogen-2-propanol kommen insbesondere 1,3-Dichlor-2-propanol und 1,3-Dibrom-2-propanol in Betracht.

Die Umsetzung der Verbindungen (III) zur Herstellung der Ausgangsverbindung der Formel (II) werden zweckmäßigerweise bei Temperaturen von 0 bis 120°C unter Normaldruck oder in einem geschlossenen Gefäß unter erhöhtem Druck durchgeführt. Als Lösungs- oder Verdünnungsmittel werden zweckmäßigerweise ein niederes aliphatisches Keton, wie Aceton, Methyläthylketon, ein niederer Alkohol mit 1 bis 4 C-Atomen, wie Methanol, Äthanol, Propanol oder Butanol, ein niederer aliphatischer oder cyclischer Äther, wie Diäthyläther, Tetrahydrofuran oder Dioxan, ein Dialkylformamid, wie Dimethylformamid oder Diäthylformamid, und Hexamethylphosphorsäuretriamid oder überschüssiges Alkylierungsmittel verwendet.

Bevorzugt werden diese Umsetzungen in Gegenwart einer Base als säurebindendes Mittel durchgeführt. Geeignete Basen sind Alkalimetallcarbonate, -hydrogencarbonate, -hydroxide, -hydride oder -alkoholate, insbesondere des Natriums und Kaliums, basische Oxide wie Aluminiumoxid oder Calciumoxid, organische tertiäre Basen, wie Pyridin, oder niedere Trialkylamine, wie Trimethyl- oder Triäthylamin, oder Piperidin. Dabei können die Basen im Verhältnis zum eingesetzten Alkylierungsmittel in katalytischer Menge oder in stöchiometrischer Menge bzw. in geringem Überschuß verwendet werden.

Bevorzugt werden die (2-Hydroxy-styryl)-isoxazole mit Epibromhydrin oder 1,2-Dibrompropanol-2 in einem polaren, aprotischen Lösungsmittel, insbesondere Dimethylsulfoxid, in Gegenwart von mindestens einem Moläquivalent Base, insbesondere Natriumhydrid, bezogen auf das Alkylierungsmittel, bei Temperaturen von 0 bis 50°C umgesetzt.

Die Ausgangsverbindungen der Formel (III) lassen sich herstellen

1. durch Umsetzung eines Phosphonsäureesters der allgemeinen Formel (IV)

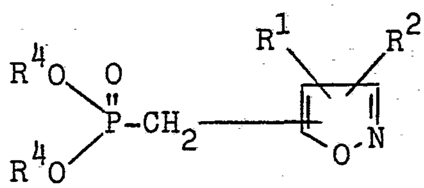

(IV)

in der $R^1$ und $R^2$ die für Formel (I) angegebenen Bedeutungen haben und $R^4$ für einen niederen Alkylrest mit 1 bis 3 C-Atomen steht, mit einem subsituierten Salicylaldehyd der allgemeinen Formel (V),

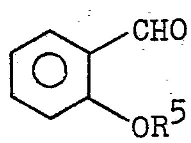

(V)

in der $R^5$ einen Alkoxyrest der Formel (VI)

$-CH-R^6$
$OR^7$

(VI)

in der $R^6$ und $R^7$ gleich oder verschieden sind und einen niederen Alkylrest mit 1 bis 3 C-Atomen darstellen, wobei $R^6$ auch Wasserstoff und $R^5$ den Tetrahydropyranylrest bedeuten können, unter den an sich üblichen Bedingungen der Wittig-Horner-Reaktion zu einer Verbindung der allgemeinen Formel (VII) und nachfolgender Abspaltung der Schutzgruppe $R^5$ unter sauren Bedingungen.

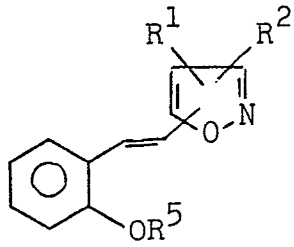

(VII)

Eine analoge Reaktion wird in der DE-OS 28 18 999 beschrieben. Die Phosphonester der allgemeinen Formel (IV) werden nach an sich bekannten Methoden aus den entsprechenden Halogenmethylisoxazolen durch Umsetzung mit Trialkylphosphiten bzw. Dialkylphosphiten im Sinne einer Arbusov- oder Michaelis-Becker--Reaktion, wie es beispielsweise im Houben-Weyl, Bd. 12/1, S. 433 ff, beschrieben ist, hergestellt.

Die Halogenmethylisoxazole werden nach an sich bekannten Verfahren zur Herstellung von Isoxazolderivaten erhalten, wie beispielsweise beschrieben wird von N.N Kochetkov und S.D. Sokolov, Adv. Het. Chem. 2, 365 ff (1963); Chem. Heterocycl. Compounds Band 17, Wiley, 1962 und Houben-Weyl, Bd. 10/3, 841-870.

Die Schutzgruppe $R^5$ stellt einen unter sauren Bedingungen leicht abspaltbaren, in alkalischem Milieu aber stabilen Rest dar, bevorzugt eine 1-Alkoxyalkylgruppe, wie den 1-Methoxy-äthylrest. Salicylaldehyde der allgemeinen Formel (V) sind bekannt oder können nach bekannten Methoden hergestellt werden durch Umsetzung des Phenolats vom Salicylaldehyd mit 1-Chloralkylalkyläther, wie Chlormethyl-methyläther oder α-Chloräthylmethyläther, oder durch Umsetzung von Salicylaldehyd mit Alkylvinyläthern unter saurer Katalyse. Die Synthese derartig geschützter Hydroxybenzaldehyde ist beschrieben. Als Beispiele seien die folgenden Literaturstellen zitiert: A. Bellino, Ann.

Chimica 48, 706-722 (1958); P. Venturella, I. Mir., J. Chem. Soc. (London), Sect. C. 1967, 411; DE-OS 22 09 128 und A. Arcoles et al Ann. Chimica 47, 667-74 (1957).

Die Umsetzung einer Verbindung der allgemeinen Formel (V) mit einem Phosphonester der allgemeinen Formel (IV) erfolgt zweckmäßigerweise in einem geeigneten Lösungsmittel in Gegenwart einer Base nach an sich üblichen Bedingungen, wie es beispielsweise in Houben-Weyl, Bd. 5/1d, S. 127 ff, und A. Maercker, Org. Reactions, Bd. 14, S. 270 ff beschrieben ist. Als inerte Lösungsmittel kommen aromatische Kohlenwasserstoffe, wie Benzol, Toluol oder Xylol, höhersiedende aliphatische oder cycloaliphatische Äther, wie Äthylenglykoldimethyläther, Tetrahydrofuran oder Dioxan, Dimethylformamid oder Dimethylsulfoxid oder Mischungen der genannten Lösungsmittel in Betracht. Die Umsetzungen werden vorteilhaft bei Raumtemperatur oder bei höheren Temperaturen von 30 bis 80°C durchgeführt. Als Basen kommen Alkalimetallhydride, -amide oder -alkoholate, insbesondere die des Natriums und Kaliums, und Butyl- und Phenyllithium in Betracht.

Die Abspaltung der Schutzgruppe unter Freisetzung der Verbindung der Formel (III) aus den Verbindungen der Formel (VII) wird nach an sich bekannten Methoden durchgeführt. In der Regel wird die Verbindung der Formel (III) ohne vorherige Reinigung verseift. Die Abspaltung wird in wäßrigem, wäßrig-alkoholischem oder alkoholischem Milieu unter Verwendung einer anorganischen Säure, wie HCl oder $H_2SO_4$, oder mit Hilfe von sauren Ionenaustauschern in einem geeigneten Lösungsmittel, wie einem niederen Alkohol, Äther, Keton, Kohlenwasserstoff etc., insbesondere Metha-

nol oder Äthanol, bei Raumtemperatur oder erhöhter Temperatur durchgeführt. Gegebenenfalls können auch Wasser, Alkoholgemische als Lösungsmittel verwendet werden. Bevorzugte Lösungsmittel sind Methanol/Wasser- und Aceton/Wasser-Gemische mit einer 1 bis 10 %igen Säurekonzentration.

2. Durch Umsetzung von Phosphoryliden der allgemeinen Formel (VIII)

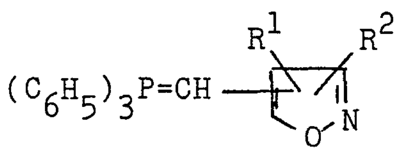

(VIII)

in der $R^1$ und $R^2$ die für Formel (I) angegebene Bedeutung haben, mit Salicylaldehyd oder einem geschützten Salicylaldehyd der allgemeinen Formel (V). Die Umsetzung erfolgt nach an sich bekannten Methoden, wie es beispielsweise im Houben-Weyl, Bd. 5/16, S. 383 ff, beschrieben wird. Bei Verwendung eines geschützten Aldehyds wird die Schutzgruppe wie unter 1 beschrieben abgespalten.

Die Verbindungen der allgemeinen Formel (III), in denen sich der Styrylsubstituent in 5-Stellung des Isoxazolringes befindet, können weiterhin hergestellt werden durch Kondensation eines geschützten Aldehyds der allgemeinen Formel (V) mit einem 5-Methylisoxazolen der allgemeinen Formel (IX),

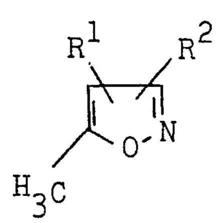

(IX)

in der $R^1$ und $R^2$ die für Formel (I) angegebenen Bedeutungen aufweisen, in Anwesenheit einer Base. Die Herstellung der Verbindungen (IX) erfolgt nach an sich bekannten Methoden, die beispielsweise in den folgenden Literaturstellen beschrieben werden: Adv. Het. Chem. 2, 365 ff (1963), Chem. Heterocycl. Comp. Bd. 17; G.T. Morgan und H. Burgess.

Die Kondensation eines 5-Methylisoxazols (IX) mit einem Aldehyd wird zweckmäßigerweise durchgeführt bei Temperaturen von -100°C bis +100°C in einem inerten Verdünnungs- oder Lösungsmittel, z.B. einem niederen Alkohol mit 1 bis 4 C-Atomen, wie Methanol, Ethanol, Propanol oder Butanol, einem niederen aliphatischen oder cyclischen Ether, wie Diethylether, Tetrahydrofuran oder Dioxan, einem N,N-Dialkylcarboxamid, wie Dimethylformamid, oder Dimethylsulfoxid oder Hexamethylphosphorsäuretriamid, einem Kohlenwasserstoff, wie Hexan oder Toluol, sowie in Mischungen derselben.

Geeignete Basen sind Alkalimetallhydroxide, -hydride, -amide oder -alkoholate, insbesondere des Natriums und Kaliums, organische Basen, wie Piperidin oder Triethylamin, Lithiumalkyle, insbesondere Methyl- und Butyllithium, sowie Lithiumamide, wie Lithium-N,N-diisopropylamid.

Die Kondensation wird bevorzugt in einem niederen Alkohol mit Natriumalkoholat bei 0 bis 80°C oder in einem aliphatischen Ether mit Li-N,N-Diisopropylamid als Base bei -80°C bis 0°C durchgeführt. Derartige Reaktionen werden beispielsweise beschrieben von G. Renzi, V. Dal Piaz und S. Pinzanti, Gazz 99, 753 (1969); A. Krishnamurthy, K.S.R. Krishna Mohan Ras und N.V. Subba Ras, Ind. J. Appl. Chem. 35, 89 (1972), US-PS 4 122 182, DE-OS 24 09 949.

Wenn die Kondensationsreaktion in einem aprotischen Lösungsmittel, wie Tetrahydrofuran durchgeführt wird, können gegebenenfalls die Benzylalkohole (X) als Zwischenprodukte

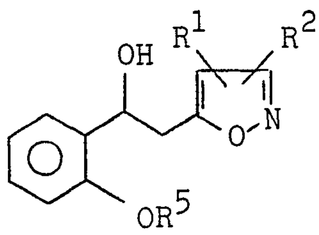

(X)

isoliert werden. Diese lassen sich nach an sich bekannten Methoden, wie sie beispielsweise in Houben-Weyl, Bd. 5/16, S. 45 ff, beschrieben sind, in die Olefine (VII) überführen.

Die Verbindungen der Formel (II), in denen A den Rest 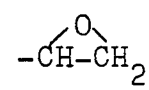 bedeutet, können auch hergestellt werden durch Umsetzung von einem Phosphonsäureester der allgemeinen Formel (IV) oder Phosphoryliden der Formel (VIII) mit einem o-Glycidylbenzaldehyd der Formel (XI)

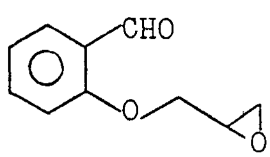

(XI)

in einer Wittig-Horner- oder Wittig-Reaktion unter den oben genannten Bedingungen. Die Reaktion einiger Phosphonsäureester mit dem Aldehyd (XI) wird beispielsweise in der DE-OS 26 23 313 beschrieben. Der Aldehyd (XI) ist bekannt und kann wie in DE-OS 26 23 313 und J. Chem. Soc. 1954, 1571, beschrieben, aus Salicylaldehyd und Epichlorhydrin unter Basenkatalyse erhalten werden.

Gemäß einem weiteren Herstellungsverfahren werden die erfindungsgemäßen Verbindungen der allgemeinen Formel (I) durch Alkylierung einer Verbindung der Formel (III) mit einer Verbindung der allgemeinen Formel (XII) oder (XIII),

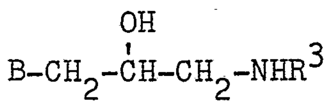

(XII)

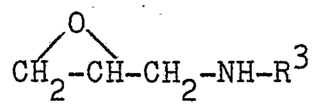

(XIII)

in denen B die für Formel (II) und $R^3$ die für Formel (I) angegebenen Bedeutungen haben, zweckmäßigerweise in Gegenwart eines Lösungsmittels und gegebenenfalls in Gegenwart eines säurebindenden Mittels bei Temperaturen von 40 bis 120°C in an sich üblicher Weise hergestellt und gegebenenfalls die erhaltene Verbindung in das Säureadditionssalz einer physiologisch verträglichen Säure überführt.

Diese Umsetzung kann beispielsweise gemäß den in der Schweizer Patentschrift 451 115 oder den in der DE-OS 22 007 751 beschriebenen Bedingungen erfolgen.

Die Alkylierung eines Phenols der Formel (III) mit einer Verbindung der Formel (XII) wird bevorzugt unter den für die Reaktion von (III) mit Epibromhydrin oder 1,2-Dibrompropanol-2 beschriebenen Bedingungen durchgeführt.

Weiterhin werden die erfindungsgemäßen Verbindungen (I) hergestellt, indem man einen Phosphonsäureester der Formel (IV) mit einer Verbindung der Formel (XIV)

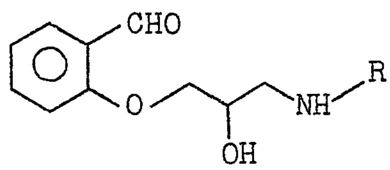 (XIV)

in der R die für Formel (I) angegebenen Bedeutungen hat, in Gegenwart einer Base und zweckmäßigerweise in Gegenwart eines Lösungsmittels in an sich üblicher Weise unter den oben beschriebenen Bedingungen der Wittig-Horner-Reaktion umsetzt.

Diese Reaktionen können beispielsweise gemäß den in der DE-OS 19 39 809 beschriebenen Bedingungen erfolgen. Die Herstellung des Aldehyds (XIV) wird in der DE-OS 22 37 228 und DE-OS 23 27 270 beschrieben.

Die erfindungsgemäßen Verbindungen (I), in denen sich der Styrylsubstituent in der 5-Position des Isoxazols befindet, lassen sich weiterhin herstellen durch Kondensation der 5-Methylisoxazole (IX) mit den Aldehyden (XIV) gemäß den oben angegebenen Bedingungen.

Bei der Herstellung fallen die erfindungsgemäßen Verbindungen als Gemische aus cis- und trans-Isomeren an, wobei in der Regel die trans-Verbindungen überwiegen. Die Isomeren lassen sich durch Kristallisation oder Säulenchromatographie ohne Schwierigkeiten voneinander abtrennen. Weiterhin besitzen die Verbindungen der Formel (I) am Kohlenstoffatom 2 der aliphatischen Seitenkette ein Chiralitätszentrum und werden als Racemate erhalten, die durch bekannte Methoden, beispielsweise durch Bildung diastereomerer Salze mit optisch aktiven Hilfssäuren wie Dibenzoylweinsäure, Campher-10-sulfonsäure, Ditoluylweinsäure oder 3-Brom-campher-8-sulfonsäure, in die optisch aktive Antipoden getrennt werden können.

Gegebenenfalls werden die erhaltenen erfindungsgemäßen Verbindungen in das Säureadditionssalz einer physiologisch verträglichen Säure überführt. Als übliche physiologisch verträgliche organische oder anorganische Säuren kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milschsäure, Weinsäure, Äpfelsäure, Zitronensäure, Salicylsäure, Adipinsäure oder Benzoesäure oder können aus Fortschritte der Arzneimittelforschung Band 10, Seite 224 bis 225, Birkhäuser Verlag, Basel und Stuttgart, 1966, oder dem Journal of Pharmaceutical Sciences, Volume 66, Seiten 1 bis 5 (1977), entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Äthanol oder Propanol, oder einem niederen Keton, wie Aceton, Methyläthylketon oder Methylisobutylketon, oder einem Äther, wie Diäthyläther, Tetrahydrofuran oder Dioxan, erhalten. Zur besseren Kristallabscheidung können auch Mischungen der genannten Lösungsmittel verwendet werden.

Die erfindungsgemäßen Verbindungen und ihre physiologisch verträglichen Säureadditionssalze sind pharmakodynamisch als aktive ß-Sympatholytika mit akut blutdrucksenkender Wirkung zu charakterisieren.

Aufgrund dieser Effekte sind die Verbindungen pharmakotherapeutisch besonders zur Behandlung der Hypertonie, der coronaren Herzkrankheit (Angina pectoris) und von Herzrhythmusstörungen geeignet.

Die Untersuchungen der pharmakodynamischen Eigenschaften wurde nach folgenden Methoden durchgeführt:

ß-sympatholytische Wirkung:
An despinalisierten Ratten (Stamm: Sprague Dawley, männlich, Gewicht 200 bis 240 g) verursacht Isoproterenol (0,1 µg/kg intravenös) Herfrequenzsteigerungen um durchschnittlich 121 ± 2,1 $min^{-1}$ bei einer Ausgangsfrequenz von 268 ± 3,9 $min^{-1}$ (Anzahl der Tiere N = 100). ß-Sympatholytika hemmen diese Frequenzsteigerungen spezifisch und dosisabhängig. Die Applikation der getesteten Substanzen erfolgt 5 min vor der Isoproterenolgabe.

Als ED 50 % werden die Dosen ermittelt, welche die Isoproterenol-Tachycardie um 50 % hemmen.

Blutdrucksenkende Wirkung an der narkotisierten Ratte:
Zur Feststellung der blutdrucksenkenden Wirkung erhalten männliche Ratten (Stamm: Sprague Dawley, Gewicht: 230 bis 280 g) in Urethan-Narkose (1,78 g/kg intraperitoneal) die Prüfsubstanzen intravenös appliziert. Die Messung des Blutdruckes in der Aorta carotis erfolgt über Statham-Transducer.

Als ED 20 % wird die Dosis ermittelt, welche den mittleren Carotisdruck um 20 % senkt.

Akute Toxizität an der Maus:
Zur Bestimmung der akuten Toxizität (LD 50) werden die Substanzen Gruppen von je 10 weiblichen NMRI-Mäusen (Gewicht: 20 bis 25 g) intraperitoneal appliziert.

Als Referenzsubstanz dient das bekannte ß-Sympatholytikum Propranolol.

Die erfindungsgemäßen Substanzen (Tab. 1) sind hochaktive ß-Sympatholytika, welche die Wirksamkeit von Propranolol übertreffen (ED 50 %). Außer der ß-sympatholytischen Wirkung besitzen sie im Gegensatz zu Propranolol eine dosisabhängige blutdrucksenkende Wirkung, die an Ratten nach intravenöser Applikation in Dosen (ED 20 %) zwischen 0,464 und 1,95 mg/kg auftritt. Propranolol senkt den Blutdruck bis zu einer Dosis von 2,15 mg/kg nicht. Erst nach der subletalen Dosis von 4,64 mg/kg wird eine Senkung von durchschnittlich 36 % beobachtet.

Die Toxizität der Substanzen ist gering. Die letalen Dosen (LD 50) sind 12 100 bis 75 000 mal höher als die ß-sympatholytisch wirksamen (ED 50%). Damit wird eine höhere therapeutische Breite erreicht als beim Propranolol (8 500).

Tabelle 1

| Substanz Beispiel Nr. | ß-Sympatholytische Wirkung Ratte i.v. ED 50 % 1) | Blutdrucksenkende Wirkung Ratte i.v. ED 20 % | Toxizität Maus i.p. LD 50 | Therapeutische Breite 2) |
|---|---|---|---|---|
| 3 | 0,00640 | 1,00 | 121 | 18900 |
| 4 | 0,00742 | 1,00 | 100 | 13500 |
| 9 | 0,01 | 0,464 | 121 | 12100 |
| 17 | 0,00681 | 1,95 | 511 | 75000 |
| 16 | 0,01 | 1,00 | 147 | 14700 |
| Propranolol | 0,0127 | - | 108 | 8500 |

1) mg/kg

2) $\frac{\text{LD } 50}{\text{ED } 50\ \%}$

Gegenstand der vorliegenden Erfindung sind demnach auch therapeutische Mittel oder Zubereitungen, die neben üblichen Träger- und Verdünnungsmitteln eine Verbindung der Formel (I) als Wirkstoff enthalten, sowie die Verwendung der neuen Verbindungen zu therapeutischen Zwecken.

Die therapeutische Mittel bzw. Zubereitungen werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmittel, wie Dextrose, Zucker, Sorbit, Mannit, Polyvinylpyrrolidon, Sprengmittel wie Mais, Stärke oder Alginsäure, Bindemitteln wie Stärke oder Gelatine, Gleitmitteln wie Magnesiumstearat oder Talk und/oder Mitteln zur Erzielung des Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmacksverbessernde Mittel wie Saccharin, Cyclamat oder Zucker sowie z.B. Aromastoffe wie Vanillin oder Orangenextrakt, enthalten. Sie können außerdem Suspendierhilfsstoffe wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einm inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten, herstellen.

Als Einzeldosis der erfindungsgemäßen Verbindungen kommen 1 bis 100 mg, vorzugsweise 3 bis 50 mg, am Menschen in Betracht.

Die Erfindung wird durch die folgenden Beispiele näher erläutert.

Herstellung von Ausgangsverbindungen

Beispiel I

(3-Methoxymethyl-isoxazolyl-5)-methanphosphonsäurediethylester

150 ml (0,87 mol) Triethylphosphit wurden bei 150°C tropfenweise mit 98 g (0,67 mol) 5-Chlormethyl-3-methoxymethyl-isoxazol versetzt. Die Mischung wurde noch 5 Stunden bei dieser Temperatur gehalten, das überschüssige Triethylphosphit abgezogen und der Rückstand fraktioniert. $Kp_{0,25}$: 143-150°C, 133 g (86 %); $^1$H-NMR ($CDCl_3$): 6,3 (d, I = 3 Hz, 1 Hz), 4,5 (I, 2 H), 4,1 (quint, I = 8, 4 H), 3,3 (d, I = 22, 2 H); 3,3 (s, 3 H); 1,3 (t, I = 8, 6 H).

Analog wurden beispielsweise hergestellt:
(3-Methyl-4-chlor-isoxazolyl-5)-methanphosphonsäurediethylester, $Kp_{0,6}$ 127°C, Ausb. 47 %
(3,5-Dimethyl-isoxazolyl-4)-methanphosphonsäurediethylester, Fp 52,7°C, Ausb. 79 %
(5-Methyl-isoxazolyl-3)-methanphosphonsäurediethylester, $Kp_{0,3}$ 122-128°C, Ausb. 70 %
(3-Carbethoxy-isoxazolyl-5)-methanphosphonsäurediethylester, $Kp_{0,01}$ 130-136°C, Ausb. 79 %

Beispiel II

3-(2-Hydroxystyryl)-5-methyl-isoxazol

Zu 48 g (0,167 mol) NaH in 150 ml Dimethylsulfoxid wurden bei RT in 10 min 36,7 g (0,15 mol) (5-Methyl-isoxazolyl--3)-methanphosphonsäureethylester und nach 1 Stunde 28,5 g (0,158 mol) 2-(1-Methoxyethoxy)-benzaldehyd getropft. Die Reaktionsmischung wurde auf 1000 ml 20 % NaCl-Lösung gegossen und mit Ether extrahiert. Der durch Abziehen des Ethers erhaltene Rückstand wurde in 100 ml Methanol gelöst. Nach Ansäuern der Lösung mit 2 n HCl fiel ein ockerfarbener Niederschlag aus, der abgesaugt und aus Aceton/Cyclohexan umkristallisiert wurde. Fp. 175°C, Ausb. 17,5 g (55 %), $^1$H-NMR ($CDCl_3$): 7,8-6,9 (m, 6 H); 6,3 (s, 1 H); 2,5 (s, 3 H).

Auf die gleiche Weise wurden unter anderem erhalten:
3-Methoxymethyl-5-(2-hydroxystyryl)-isoxazol, Fp. 155°C, Ausb. 48 %.
4-Chlor-5-(2-hydroxystyryl)-3-methyl-isoxazol, Fp. 185°C, Ausb. 54 %,
3,5-Dimethyl-4-(2-hydroxystyryl)-isoxazol, Fp. 153°C, Ausb. 47 %.

<u>Beispiel III</u>
5-(2-Hydroxystyryl)-4-nitro-3-methyl-isoazol

82,3 g (0,457 mol) 2-(1-Methoxy-ethoxy)-benzaldehyd wurden zu einer Lösung von 65,3 g (0,457 mol) 3,5-Dimethyl-4--nitroisoxazol in 1000 ml Methanol/20 ml Piperidin getropft und 15 min unter Rückfluß erhitzt. Die beim Abkühlen ausfallenden Kristalle wurden abgesaugt und in 3,5 l siedendem Methanol suspendiert. Nach dem Ansäuern mit 5 n HCl entstand eine klare Lösung, aus der beim Abkühlen ein gelber Niederschlag ausfiel. Dieser wurde abgesaugt und getocknet. Fp. 247-250°C, Ausb. 96 g (90 %); $^1$H-NMR ($CDCl_3$): 7,9 (d, I = 9,1 H); 7,75 (d, I = 9,1 H); 7,6 (d, I = 4, 1 H); 7,3 (t, I = 4,1 H); 6,95 (d, I = 4, 1 H); 6,85 (t, I = 4,1 H); 2,5 (s, 3 H)

| | | | | |
|---|---|---|---|---|
| $C_{12}H_{10}N_2O_4$ (246,2) | Ber. | 58,5 C | 4,1 H | 11,4 N |
| | Gef. | 58,5 C | 4,2 H | 11,5 N |

Beispiel IV

3-Chlor-5-(2-hydroxystyryl)-isoxazol

10,5 g (98 mol) 3-Chlor-5-methylisoxazol wurden in 100 ml THF gelöst und bei -70°C mit 95 mmol n-BuLi (1,6 n in Hexan) versetzt. Nach 30 min wurden 16 g (98 mmol) 2-(1--Methoxymethoxy)-benzaldehyd zugetropft, noch 15 min bei -50°C gerührt, mit 3 ml $H_2O$ versetzt, die Mischung auf RT aufgewärmt und auf Eiswasser gegossen. Es wurde mit Ether extrahiert, die organische Phase abgetrennt, getrocknet und einrotiert.

Der Rückstand (22,5 g) wurde in 150 ml $CH_2Cl_2$/70 ml Triethylamin gelöst und bei 0°C mit 18,5 g (162 mmol) Methansulfonylchlorid versetzt. Die Suspension wurde 12 Stunden bei RT gerührt und auf 300 ml Eiswasser gegossen. Abtrennen, Trocknen und Einengen der $CH_2Cl_2$-Phase ergab einen öligen Rückstand, der in 40 ml Methanol gelöst wurde. Nach Ansäuern mit 2 n HCl wurde mit Wasser versetzt, bis ein Niederschlag auszufallen begann. Dieser wurde nach 1 Stunde abgesaugt und getrocknet. Fp. 183-186°C, Ausb.: 9,4 g (51 %), $^1H$-NMR ($CDCl_3$): 7,6-6,5 (m, 6 H); 6,2 (s, 1 H).

Beispiel V

3-Carbethoxy-5[2-(2,3-epoxypropoxy)-styryl]-isoxazol

Eine Suspension von 24 mmol NaH (55 % in Paraffinöl) in 80 ml DMF wurde bei RT tropfenweise mit einer Lösung von 7,0 g (24 mmol) (3-Carbethoxy-isoxazol-5)-methanphosphonsäurediethylester und 4,3 g (24 mmol) 2-(2,3-Epoxypropoxy)--benzaldehyd in 40 ml DMF versetzt und über Nacht bei RT gerührt. Die Reaktionsmischung wurde auf 200 ml Eiswasser gegossen, mit Ether extrahiert, die Etherphasen getrocknet und einrotiert. Umkristallisation des Rückstandes aus Ether/Hexan ergab 3,2 g (44 %). Fp. 91-93°C, $^1H$-NMR

5-[2-(2,3-Epoxypropoxy)-styryl]-3-methoxymethyl-isoxazol, Ausb. 90 %. Die Substanz wurde roh weiter umgesetzt.

4-Chlor-5-[2-(2,3-epoxypropoxy)-styryl]-3-methylisoxazol, Ausb. 65 %

3-[2-(2,3-Epoxypropoxy)-styryl]-5-methylisoazol, Ausb. 87 %, Fp. 81,2°C

| | | | | |
|---|---|---|---|---|
| $C_{15}H_{15}O_3N$ (257) | Ber. | 70,0 C | 5,9 H | 5,4 N |
| | Gef. | 69,6 C | 5,8 H | 5,5 N |

Herstellung von erfindungsgemäßen Verbindungen

Beispiel 1

3-Chlor-5-[2-(2-hydroxy-3-tert.butylamino-propoxy)-styryl]-isoxazol

4,8 g (17 mmol) 3-Chlor-5-[2-(2,3-epoxypropoxy)-styryl]-isoxazol werden mit 150 ml Isopropanol/10 ml tert.-Butylamin 3 Stunden bei Raumtemperatur gerührt. Der nach dem Abdestillieren des Lösungsmittels erhaltene Rückstand wird aus Ether umkristallisiert. 3,8 g (63 %), Fp. 94-97°C.

| | | | | | |
|---|---|---|---|---|---|
| $C_{18}H_{23}N_2O_3Cl$ (350) | Ber. | 61,6 C | 6,6 H | 8,0 N | 10,1 Cl |
| | Gef. | 61,5 C | 6,6 H | 7,9 N | 10,1 Cl |

Analog werden die Verbindungen der Beispiele 2 bis 26 erhalten und gegebenenfalls in ein Salz überführt:

($CDCl_3$): 7,5-6,7 (m, 6 H); 6,6 (s, 1 H); 4,6-3,8 (m + q, J = 7, 4 H); 3,4 (m 1 H); 2,8 (m, 2 H); 1,4 (t, J = 7,3 H).

Beispiel VI

3,5-Dimethyl-4-[2-(2,3-epoxypropoxy)-styryl]-isoxazol

Eine Mischung von 11,0 g (51 mmol) 3,5-Dimethyl-4-(2--hydroxystyryl)-isoxazol, 10,5 g (76 mmol) Epibromhydrin und 141 g (102 mmol) $K_2CO_3$ in 50 ml Dimethylformamid wurde 2,5 Stunden bei 60°C gerührt, bei 15°C unter Rühren mit 150 ml Wasser versetzt, der dabei ausgefallene Niederschlag abgesaugt und aus Ethanol umkristallisiert. 11,5 g (83 %), Fp. 90,3°C

| | | | | |
|---|---|---|---|---|
| $C_{16}H_{17}NO_3$ (271) | Ber. | 70,8 C | 6,3 H | 5,2 N |
| | Gef. | 70,6 C | 6,2 H | 5,2 N |

Analog werden erhalten:

3-Chlor-5-[2-(2,3-epoxypropoxy)-styryl]-isoxazol, Ausb. 85 %

3,4-Dimethyl-5-[2-(2,3-epoxypropoxy)-styryl]isoxazol, Fp. 82-84°C, Ausb. 79 %

| | | | | |
|---|---|---|---|---|
| $C_{16}H_{17}NO_3$ (271) | Ber. | 70,8 C | 6,3 H | 5,2 N |
| | Gef. | 70,7 C | 6,2 H | 5,3 N |

4-Carboxy-5-[2-(2,3-epoxypropoxy)-styryl]-3-methylisoxazol Fp. 169-172°C; Ausb. 64 %

| | | | | |
|---|---|---|---|---|
| $C_{16}H_{15}NO_2$ (301) | Ber. | 63,8 C | 5,0 H | 4,7 N |
| | Gef. | 63,9 C | 5,1 H | 4,8 N |

Beispiel 2

3-Chlor-5-[2-(2-hydroxy-3-isopropylamino-propoxy)-styryl]-isoxazol, Ausb. 74 %, Fp. 114-116°C

| | | | | | | |
|---|---|---|---|---|---|---|
| $C_{17}H_{21}N_2ClO_3$ | (336) | Ber. | 60,6 C | 6,4 H | 8,3 N | 10,5 Cl |
| | | Gef. | 60,6 C | 6,2 H | 8,4 N | 10,5 Cl |

Beispiel 3

3-Methoxymethyl-5-[2-(2-hydroxy-3-isopropylamino-propoxy)-styryl]-isoxazol
Ausb. 63 % (Hydrochlorid), Fp. 124°C

| | | | | | | |
|---|---|---|---|---|---|---|
| $C_{19}H_{27}ClN_2O_4$ | (383) | Ber. | 59,6 C | 7,1 H | 7,3 N | 9,3 Cl |
| | | Gef. | 59,1 C | 7,2 H | 7,2 N | 9,0 Cl |

Beispiel 4

3-Methoxymethyl-5-[2-(2-hydroxy-3-tert.-butylamino-propoxy)-styryl]-isoxazol
Ausb. 78 % (Hydrochlorid), Fp. 152°C

| | | | | | | |
|---|---|---|---|---|---|---|
| $C_{20}H_{29}ClN_2O_4$ | (397) | Ber. | 60,5 C | 7,4 H | 7,1 N | 8,9 Cl |
| | | Gef. | 60,4 C | 7,4 H | 7,2 N | 8,9 Cl |

Beispiel 5

3-[2-(2-Hydroxy-3-isopropylamino-propoxy)-styryl]-5-methylisoxazol
Ausb. 64 % (Hydrochlorid), Fp. 155°C

| | | | | | | |
|---|---|---|---|---|---|---|
| $C_{18}H_{25}ClN_2O_3$ | (353) | Ber. | 61,3 C | 7,1 H | 7,9 N | 10,0 Cl |
| | | Gef. | 61,1 C | 7,1 H | 8,0 N | 8,9 Cl |

Beispiel 6

3-[2-(2-Hydroxy-3-tert.-butylamino-propoxy)-styryl]-5--methylisoxazol
Ausb. 39 % (Hydrochlorid); Fp. 181°C

| | | | | | | |
|---|---|---|---|---|---|---|
| $C_{19}H_{27}ClN_2O_3$ | (367) | Ber. | 62,2 C | 7,4 H | 7,6 N | 9,7 Cl |
| | | Gef. | 61,9 C | 7,5 H | 7,8 N | 9,7 Cl |

Beispiel 7

5-[2-(2-Hydroxy-3-(3-methyl-butin-(1)-yl-3-amino)-propoxy)--styryl]-3-methoxymethyl-isoxazol
Ausb. 82 % (Hydrochlorid), Fp. 150°C

| | | | | | | |
|---|---|---|---|---|---|---|
| $C_{21}H_{27}ClN_2O_4$ | (407) | Ber. | 62,0 C | 6,7 H | 6,9 N | 8,7 Cl |
| | | Gef. | 61,9 C | 6,5 H | 7,0 N | 8,7 Cl |

Beispiel 8

5-[2-(2-Hydroxy-3-(butin-(1)-yl-3-amino)-propoxy)-styryl]--3-methoxymethyl-isoxazol
Ausb. 29 %, Fp. 105°C

| | | | | | |
|---|---|---|---|---|---|
| $C_{20}H_{24}N_2O_4$ | (356) | Ber. | 67,4 C | 6,7 H | 7,9 N |
| | | Gef. | 67,3 C | 6,7 H | 7,9 N |

Beispiel 9

5-[2-(2-Hydroxy-3-cyclopropylamino-propoxy)-styryl]-3-methoxymethyl-isoxazol
Ausb. 45 % Fp. 93°C

| | | | | | |
|---|---|---|---|---|---|
| $C_{19}H_{24}N_2O_4$ | (344) | Ber. | 66,3 C | 7,0 H | 8,1 N |
| | | Gef. | 66,4 C | 6,9 H | 8,3 N |

Beispiel 10

5-[2-(2-Hydroxy-3-(1-cyclopropyl-ethyl-1-amino)-propoxy)-styryl]-3-methoxymethyl-isoxazol
Ausb. 30 % (Fumarat), Fp. 151°C

| | | | | | |
|---|---|---|---|---|---|
| $C_{23}H_{32}N_2O_6$ (433) | Ber. | 63,9 C | 7,5 H | 22,2 O | 6,5 N |
| | Gef. | 63,9 C | 7,0 H | 22,3 O | 6,5 N |

Beispiel 11

5-[2-(2-Hydroxy-3-(2-methyl-butyl-2-amino)-propoxy)-styryl]-3-methoxymethyl-isoxazol
Ausb. 50 % (Hydrogenfumarat); Fp. 119°C

| | | | | | |
|---|---|---|---|---|---|
| $C_{25}H_{34}N_2O_8$ (491) | Ber. | 61,2 C | 7,0 H | 26,1 O | 5,7 N |
| | Gef. | 61,1 C | 7,2 H | 26,3 O | 5,7 N |

Beispiel 12

5-Methyl-3-[2-(2-hydroxy-3-cyclopropylamino-propoxy)-styryl]-isoxazol
Ausb. 72 %; Fp. 138°C

| | | | | |
|---|---|---|---|---|
| $C_{18}H_{22}N_2O_3$ (314) | Ber. | 68,8 C | 7,1 H | 8,9 N |
| | Gef. | 68,4 C | 7,1 H | 8,9 N |

Beispiel 13

5-Methyl-3-[2-(2-hydroxy-3-(3-methyl-butin-(1)-yl-3-amino)-propoxy-styryl]-isoxazol
Ausb. 89 %, Fp. 123°C

| | | | | |
|---|---|---|---|---|
| $C_{20}H_{24}N_2O_3$ (340) | Ber. | 70,6 C | 7,1 H | 8,2 N |
| | Gef. | 70,3 C | 7,3 H | 8,3 N |

Beispiel 14

5-Methyl-3-[2-(2-hydroxy-3-(2-methyl-butyl-2-amino)-propoxy)-styryl]-isoxazol
Ausb. 56 %, Fp. 101°C

| | | | | | |
|---|---|---|---|---|---|
| $C_{20}H_{28}N_2O_3$ (344) | Ber. | 69,7 C | 8,2 H | 8,1 N | |
| | Gef. | 69,6 C | 8,2 H | 8,2 N | |

Beispiel 15

5-Methyl-3-[2-(2-hydroxy-3-(1-cyclopropylethyl-1-amino)-propoxy)-styryl]-isoxazol
Ausb. 42 %; Fp. 107°C

| | | | | | |
|---|---|---|---|---|---|
| $C_{20}H_{26}N_2O_3$ (342) | Ber. | 70,2 C | 7,7 H | 8,2 N | |
| | Gef. | 70,1 C | 7,7 H | 8,2 N | |

Beispiel 16

4-Chlor-3-methyl-5-[2-(2-hydroxy-3-tert.-butylamino-propoxy)-styryl]-isoxazol
Ausb. 43 %, Fp. 109°C

| | | | | | |
|---|---|---|---|---|---|
| $C_{19}H_{25}ClN_2O_3$ (365) | Ber. | 62,6 C | 6,9 H | 7,7 N | 9,7 Cl |
| | Gef. | 62,7 C | 6,7 H | 7,7 N | 10,0 Cl |

Beispiel 17

4-Chlor-3-methyl-5-[2-(2-hydroxy-3-isopropylamino-propoxy)-styryl]-isoxazol
Ausb. 65 %, Fp. 122°C

| | | | | | |
|---|---|---|---|---|---|
| $C_{18}H_{23}ClN_2O_3$ (351) | Ber. | 61,6 C | 6,6 H | 8,0 N | 10,1 Cl |
| | Gef. | 61,9 C | 6,2 H | 8,3 N | 10,1 Cl |

Beispiel 18

3,5-Dimethyl-4-[2-(2-hydroxy-3-tert.-butylamino-propoxy)-styryl]-isoxazol
Ausb. 60 %, Fp. 200°C (Hydrochlorid)

$C_{20}H_{29}ClN_2O_3$ (380) Ber. 63,1 C 7,6 H 7,4 N
Gef. 63,1 C 7,6 H 7,5 N

Beispiel 19

3,5-Dimethyl-4-[2-(2-hydroxy-3-isopropylamino-propoxy)-styryl]-isoxazol
Ausb. 74 %, Fp. 134°C

$C_{19}H_{26}N_2O_3$ (330) Ber. 69,1 C 7,9 H 8,5 N
Gef. 69,0 C 7,9 H 8,6 N

Beispiel 20

3,4-Dimethyl-5-[2-(2-hydroxy-3-isopropylamino-propoxy)-styryl]-isoxazol
Ausb. 44 %, Fp. 196°C (Fumarat)

$C_{21}H_{28}N_2O_5$ (388) Ber. 64,7 C 7,1 H 7,2 N
Gef. 64,9 C 7,2 H 7,2 N

Beispiel 21

3,4-Dimethyl-5-[2-(2-hydroxy-3-tert.butylamino-propoxy)-styryl]-isoxazol
Ausb. 78 %, Fp. 197°C (Fumarat)

$C_{22}H_{30}N_2O_5$ (402) Ber. 65,6 C 7,5 H 7,0 N
Gef. 65,2 C 7,5 H 6,9 N

Beispiel 22

4-Carboxy-3-methyl-5-[2-(2-hydroxy-3-tert.butylamino--propoxy)-styryl]-isoxazol
Ausb. 36 %, Fp. 250°C (Hydrochlorid)

| | | | | | | |
|---|---|---|---|---|---|---|
| $C_{20}H_{27}ClN_2O_5$ | (411) | Ber. | 58,5 C | 6,6 H | 6,8 N | 8,6 Cl |
| | | Gef. | 58,4 C | 6,4 H | 7,0 N | 8,7 Cl |

Beispiel 23

4-Carboxy-3-methyl-5-[2-(2-hydroxy-3-isopropylamino-propoxy)-styryl]-isoxazol
Ausb. 21 %, Fp. 247-248°C (Hydrochlorid)

| | | | | | | |
|---|---|---|---|---|---|---|
| $C_{19}H_{25}N_2O_5$ | (397) | Ber. | 57,5 C | 6,4 H | 7,1 N | 8.9 Cl |
| | | Gef. | 57,1 C | 6,1 H | 6,8 N | 9,2 Cl |

Beispiel 24

3-Carbethoxy-5-[2-(2-hydroxy-3-tert.butylamino-propoxy)--styryl]-isoxazol
Ausb. 59 %, Fp. 185-187°C (Fumarat . $H_2O$)

| | | | | | |
|---|---|---|---|---|---|
| $C_{23}H_{32}N_2O_8$ | (504) | Ber. | 59,5 C | 6,8 H | 6,0 N |
| | | Gef. | 59,3 C | 6,5 H | 5,6 N |

Beispiel 25

3-(N-Methylcarboxamido)-5-[2-(2-hydroxy-3-tert.butylamino-propoxy)-styryl]-isoxazol
Ausb. 61 %, Fp. 114-115°C

| | | | | | |
|---|---|---|---|---|---|
| $C_{20}H_{27}N_3O_4$ | (373) | Ber. | 64,3 C | 7,3 H | 11,3 N |
| | | Gef. | 64,0 C | 7,3 H | 10,7 N |

Beispiel 26

3-(N-Methylcarboxamido)-5-[2-(2-hydroxy-3-isopropylamino-propoxy)-styryl]-isoxazol
Ausb. 54 %, Fp. 202°C (Fumarat)

| | | | | |
|---|---|---|---|---|
| $C_{21}H_{27}N_3O_6$ (418) | Ber. | 60,3 C | 6.9 H | 10,5 N |
| | Gef. | 59,9 C | 6,6 H | 9,9 N |

Beispiel 27

4-Nitro-3-methyl-5-[2-(2-hydroxy-3-tert.butylamino-propoxy)-styryl]-isoxazol

5,5 g (23 mmol) 2-(2-Hydroxy-3-tert.butylaminopropoxy)--benzaldehyd und 3,3 g (23 mmol) 4-Nitro-3,5-dimethyl-isoxazol werden in 60 ml Methanol/1 ml Piperidin bei Raumtemperatur 18 Stunden gerührt. Der Niederschlag wird abfiltriert und aus Ethanol umkristallisiert.
3,7 g (43 %), Fp. 98-102°C

| | | | | |
|---|---|---|---|---|
| $C_{19}H_{25}N_3O_5$ (375) | Ber. | 60,8 C | 6,7 H | 11,2 N |
| | Gef. | 60,4 C | 6,7 H | 11,0 N |

Beispiel 28

4-Nitro-3-methyl-5-[2-(2-hydroxy-3-isopropylamino-propoxy)-styryl]-isoxazol

Die Verbindung wird wie für Beispiel 27 beschrieben aus 2-(2-Hydroxy-3-isopropylamino-propoxy)-benzaldehyd und 4-Nitro-3,5-dimethylisoxazol hergestellt. Ausb. 26 %, Fp. 154-155°C

| | | | | |
|---|---|---|---|---|
| $C_{18}H_{23}N_3O_5$ (361) | Ber. | 59,8 C | 6,4 H | 11,6 N |
| | Gef. | 59,9 C | 6,3 H | 11,5 N |

Formulierungsbeispiele, die in üblicher Weise hergestellt werden:

1. Tabletten

a)

| | |
|---|---|
| Ein Wirkstoff der Formel I | 5 mg |
| Lactose | 200 mg |
| Methylcellulose | 15 mg |
| Maisstärke | 50 mg |
| Talkum | 11 mg |
| Magnesiumstearat | 4 mg |
| | 285 mg |

b)

| | |
|---|---|
| Ein Wirkstoff der Formel I | 20 mg |
| Lactose | 178 mg |
| Avicel | 80 mg |
| Polywachs 6000 | 20 mg |
| Magnesiumstearat | 2 mg |
| | 300 mg |

c)

| | |
|---|---|
| Verbindung der Formel I | 50 mg |
| Polyvinylpyrrolidon (mittl. M.G. 25 000) | 170 mg |
| Polyäthylenglykol (mittl. M.G. 4 000) | 14 mg |
| Hydroxypropylmethylcellulose | 40 mg |
| Talkum | 4 mg |
| Magnesiumstearat | 2 mg |
| | 280 mg |

Der Wirkstoff wird mit Polyvinylpyrrolidon in 10 %iger wäßriger Lösung befeuchtet, durch ein Sieb mit der lichten Maschenweite 1,0 mm getrieben und bei 50°C getrocknet. Dieses Granulat wird mit Polyäthylenglykol (mittl. M.G. 4 000), Hydroxypropylmethylcellulose, Talkum und

Magnesiumstearat vermischt und zu Tabletten à 280 mg verpreßt.

2. Beispiel für Dragees

| | |
|---|---|
| Verbindung der Formel I | 3 mg |
| Lactose | 90 mg |
| Maisstärke | 60 mg |
| Polyvinylpyrrolidon | 6 mg |
| Magnesiumstearat | 1 mg |
| | 160 mg |

Die Mischung der Wirkstoffsubstanz mit Lactose und Maisstärke wird mit einer 8 %igen wäßrigen Lösung des Polyvinylpyrrolidons durch Sieb 1,5 mm granuliert, bei 50°C getrocknet und nochmals durch Sieb 1,0 mm gerieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

3. Kapselformulierung

| | |
|---|---|
| Verbindung der Formel I | 5,0 mg |
| Magnesiumstearat | 2,0 mg |
| Milchzucker | 19,3 mg |

4. Injektionslösung

| | |
|---|---|
| Verbindung der Formel I | 10 mg |
| Natriumchlorid | 9 mg |
| destilliertes Wasser, q.s. auf 1,0 ml | |

Patentansprüche

1. Verbindungen der allgemeinen Formel I

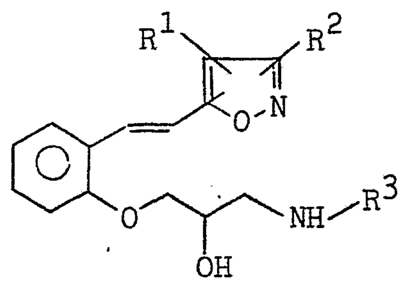

(I)

in der

$R^1$ und $R^2$ gleich oder verschieden sind und ein Wasserstoffatom, einen Alkylrest mit 1 bis 6 C-Atomen, der gegebenenfalls durch Hydroxy, Amino, Monoalkylamino, Dialkylamino oder Alkoxy mit jeweils 1 bis 3 C-Atomen im Alkyl substituiert ist, eine Nitro-, Cyano-, Carboxy-, Carbalkoxy-, Carboxamido-, Carboxalkylamidogruppe mit jeweils 1 bis 3 C-Atomen im Alkyl, ein Halogenatom, eine Hydroxy-, Amino-, Alkylamino-, Dialkylamino- oder Alkoxygruppe mit jeweils 1 bis 3 C-Atomen im Alkyl bedeuten, ausgenommen Verbindungen, bei denen im Isoxazolring der Styrylrest sich in 5-Stellung befindet und in 4-Stellung ein Wasserstoffatom und in 3-Stellung ein Alkylrest mit 1 bis 4 C-Atomen steht, und

$R^3$ ein Wasserstoffatom, einen Alkylrest mit 1 bis 8 C-Atomen, der gegebenenfalls durch Hydroxy, Alkoxy mit 1 bis 3 C-Atomen im Alkyl oder einen Cycloalkylrest mit 3 bis 8 C-Atomen im Ring substituiert ist, einen Alkenyl- oder Alkinylrest mit 2 bis 8 C-Atomen, einen Cycloalkylrest mit 3 bis 8 C-Atomen im Ring, wobei gegebenenfalls der Cycloalkylring ein- oder

zweifach durch einen Alkylrest mit 1 bis 3 C-Atomen substituiert ist, darstellt, und ihre physiologisch verträglichen Säureadditionssalze.

2. Verbindungen der Formel I nach Anspruch 1, in denen $R^1$ und $R^2$ ein Wasserstoffatom, ein Chloratom, eine Methyl-, Äthyl- oder Methoxymethylgruppe, $R^3$ einen Alkylrest mit 3 bis 6 C-Atomen, 3-Methyl-1-butin-3-yl oder einen Cyclopropylrest bedeuten und der Isoxazolring in 5-Stellung durch den Styrylrest substituiert ist, ausgenommen von Verbindungen, bei denen im Isoxazolring in 4-Stellung ein Wasserstoffatom und in 3-Stellung ein Alkylrest mit 1 bis 4 C-Atomen steht, und ihre physiologisch verträglichen Säureadditionssalze.

3. Verfahren zur Herstellung von Verbindungen der Formel I nach Anspruch 1, dadurch gekennzeichnet, daß man einen Phenoläther der Formel II

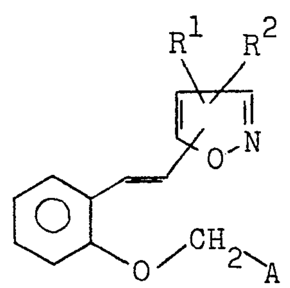

(II)

in der $R^1$ und $R^2$ die für Formel I angegebenen Bedeutungen haben und A den Rest

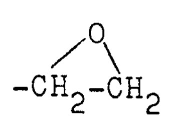 oder $-CH(OH)-CH_2-B$

worin B für eine nukleofuge Abgangsgruppe steht, bedeutet, mit einem Amin der allgemeinen Formel

$$H_2N-R$$

in der R die für $R^3$ in Formel I angegebenen Bedeutungen hat, zweckmäßigerweise in einem Lösungsmittel und gegebenenfalls in Gegenwart eines säurebindenden Mittels in an sich bekannter Weise umsetzt und gegebenenfalls die erhaltene Verbindung in ein Säureadditionssalz einer physiologisch verträglichen Säure überführt.

4. Therapeutisches Mittel, gekennzeichnet durch einen Gehalt einer Verbindung der Formel I oder ihres physiologisch verträglichen Säureadditionssalzes als Wirkstoff neben üblichen Trägerstoffen und Verdünnungsmitteln.

Europäisches Patentamt

# EUROPÄISCHER RECHERCHENBERICHT

Nummer der Anmeldung
EP 81 10 0948

| EINSCHLÄGIGE DOKUMENTE | | | KLASSIFIKATION DER ANMELDUNG (Int Cl ) |
|---|---|---|---|
| Kategorie | Kennzeichnung des Dokuments mit Angabe, soweit erforderlich, der maßgeblichen Teile | betrifft Anspruch | |
| D | EP - A - 0 005 192 (BASF)<br>* Ansprüche 1,8,16 *<br>& DE - A - 2 818 999<br>-- | 1-4 | C 07 D 261/08<br>261/10<br>261/18<br>A 61 K 31/42//<br>C 07 F 9/65<br>C 07 D 401/12 |
| | DE - A - 1 939 809 (KARL THOMAE)<br>* Ansprüche 1,6,7,21 *<br>-- | 1,4 | RECHERCHIERTE SACHGEBIETE (Int. Cl.³) |
| A | US - A - 3 624 079 (DANIEL LEDNICER)<br>* Ansprüche 1; Spalten 4,5 *<br>---- | 1,4 | C 07 D 261/08<br>261/10<br>261/18 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt | | |

| Recherchenort | Abschlußdatum der Recherche | Prüfer |
|---|---|---|
| Den Haag | 25-05-1981 | HENRY |